# EUROPEAN PATENT SPECIFICATION

(11) **EP 2 144 901 B1**
(45) Date of publication and mention of the grant of the patent: **16.03.2011**
(21) Application number: 08774046.0
(22) Date of filing: 07.05.2008
(51) Int. Cl.: C07D 409/14, C09K 11/06

(54) **OLIGOTHIOPHENE DERIVATIVES**
OLIGOTHIOPHENDERIVATE
DÉRIVÉS D'OLIGOTHIOPHÈNE

(30) Priority: 08.05.2007 EP 07447030
(43) Date of publication of application: 20.01.2010
(73) Proprietor: Université Libre de Bruxelles, 1050 Bruxelles (BE); Université de Mons, 7000 Mons (BE)
(72) Inventor: LEROY, Julie, B-1080 Bruxelles (BE); SERGUEEV, Sergeui, B-1160 Bruxelles (BE); DIDIER, Delphine, B-1050 Bruxelles (BE); GEERTS, Yves, B-1160 Bruxelles (BE); CORNIL, Jérôme, B-6120 Nalinnes (BE)
(74) Representative: pronovem
(86) International application number: PCT/EP2008/055643
(87) International publication number: WO 2008/135592

(56) References cited:
- JP-A- 2003 062 274
- US-A1- 2002 072 618
- DATABASE CA [Online] CHEMICAL ABSTRACTS SERVICE, COLUMBUS, OHIO, US; KIM, DONG-HA ET AL: "Synthesis of silicon-bridged oligothiophenes and applications to thin film transistors" XP002455035 retrieved from STN Database accession no. 2006:308004 & CHEMISTRY LETTERS , 35(3), 266-267 CODEN: CMLTAG; ISSN: 0366-7022, 2006,
- DATABASE CA [Online] CHEMICAL ABSTRACTS SERVICE, COLUMBUS, OHIO, US; KIM, HYUNG-SUN ET AL: "Synthesis and characterization of new thiophene derivative containing alkylene linkage" XP002455036 retrieved from STN Database accession no. 2003:658042 & POLYMER PREPRINTS (AMERICAN CHEMICAL SOCIETY, DIVISION OF POLYMER CHEMISTRY) , 44(2), 423-424 CODEN: ACPPAY; ISSN: 0032-3934, 2003,
- SATO T ET AL: "Synthesis and Photophysical Properties of 1,3-Di(oligothienyl)propane" TETRAHEDRON LETTERS, ELSEVIER, AMSTERDAM, NL, vol. 38, no. 34, August 1997 (1997-08), pages 6039-6042, XP004086566 ISSN: 0040-4039 cited in the application
- A. BERLIN AND CO: "Anodic coupling of oligothiophenes bridged by ethylene groups" SYNTHETIC METALS, vol. 55, 1993, pages 4796-4801, XP002455032 cited in the application

## Description

### Field of the invention

The present invention relates to new bridged oligothiophene derivatives and to their use in a liquid crystal medium for electronic devices, in particular for semiconductors.

### Technical background of the invention and prior art

The transport of electric charges in a field effect transistor occurs in the first molecular layers of the interlayer situated between the dielectric layer and the semi-conductive layer. When said first molecular layers are interrupted by some defects, the conductivity of those layer decreases. These defects are one of the reasons for the poor reproducibility of the threshold voltage of those transistors.

In the past, this problem was handled via the highest possible flatness of the dielectric layer; but on a microscopic scale, surfaces are never perfectly flat.

Recently, this problem was approached by self-aligning molecules. This self alignment can again be perturbed by surface irregularities at molecular scale.

The approach of the present invention is to provide molecules that are able to transport charges in three dimensions instead of two dimensions where two conjugated segments are connected by a σ-bond bridge. The latter promotes charge transport that is less sensitive to surface defects.

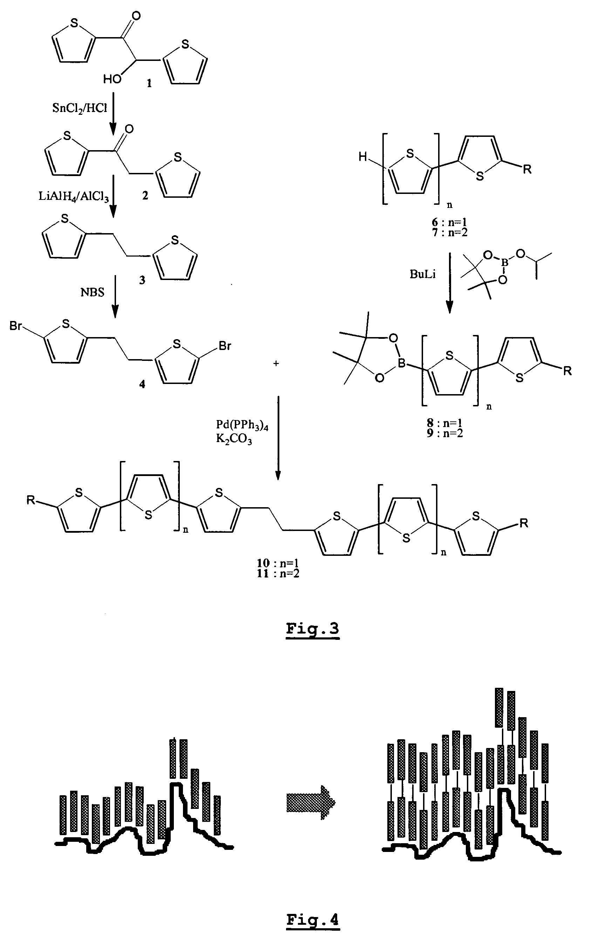

Figure 4 of the present invention shows the principle of the improvement reached by a bridged molecule compared to a non-bridged molecule.

The bridged molecules of the present invention are oligothiophene derivatives.

Bridged oligothiophene derivatives have been disclosed by A.Berlin et al, "Anodic coupling of oligothiophenes bridged by ethylene groups" in Synthetic Metals, 55-57 (1993), 4796-4801. Similar structures were also disclosed by Tadatake Sato and al., "Synthesis and photophysical properties of 1,3-Di(oligothienyl)propane" in Tetrahedron Letters, Vol.38, N°34, pp.6039-6042, 1997.

Nevertheless, the molecules disclosed in the above-cited references are different from those of the present invention and are synthesised for other purposes.
Kim DONG et al. in "Synthesis of silicon-bridged oligothiophenes and applications to thin film transistors" disclose the synthesis of mono- and disilanylene-bridged oligothiophenes and their TFT properties as p-type semiconductors. Among them, best TFT performance was allegedly achieved by using bis(ethylquinquethienyl)dibutylsilane as the active compound. US-A1-2002/0072618 discloses soluble derivatives of un-bridged sexithiophene in which terminal carbons are substiturted with various polar groups, Kim HYONG-SUN et al. in "Synthesis and characterization of new thiophene derivative containing alkylene linkage" disclose the synthesis and the characterization of oligothiophenes having alkylene linkages by sequential Pd catalyzed cross-coupling reactions of substituted thiophenes as well as their thermal properties. Also, 1,2-Bis[2'-hexyl[2,2':5'2":5",2"] quater-thiophenyl]hexane (BH4TH) has been described as allegedly being highly soluble and as having liquid crystal-like structure which would render them of interest for use as semiconductors in e.g., field-effect transistors for electronics applications.

### Aims of the invention

The present invention aims to provide new molecular structures based on bridged oligothiophene derivatives that are able to overcome the drawbacks of the prior art and able to reduce impact of the surface defects due to irregular surfaces on which the semi-conductive layer is applied.

### Summery of the invention

The present invention discloses an oligothiophene derivative of the formula I or II: 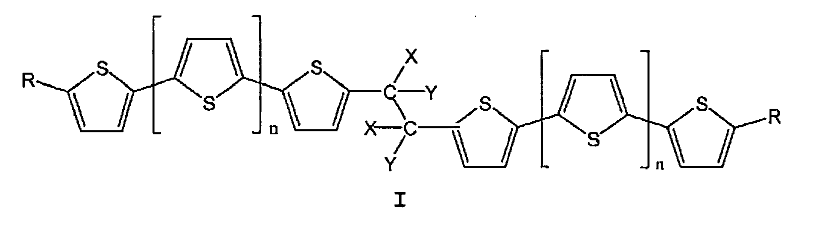 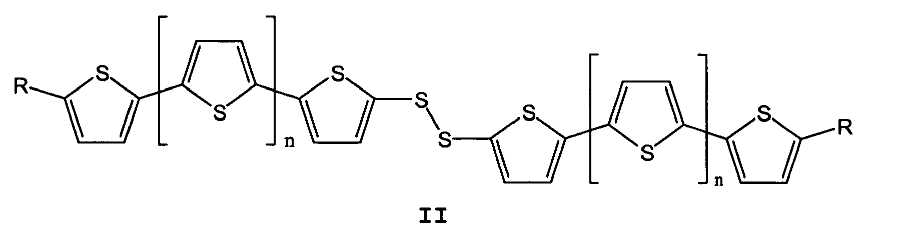 wherein
X = H or CH₃,
Y = H or CH₃,
n = 1 or 2, and
R = carbon chain, branched or not, saturated or not, with 6 to 15 carbon atoms.

Preferred embodiments of the present invention are:
- the oligothiophene derivatives of the formula I or II wherein R is a, b, or c with:
   **a :** 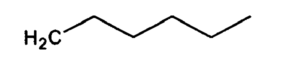
   **b :** 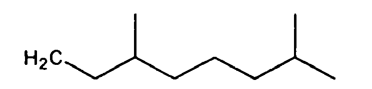
   **c :** 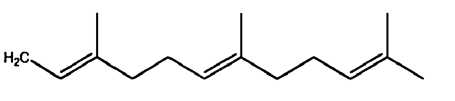
- the oligothiophene derivative of the formula I, wherein X = Y = H and wherein R is a and n = 1.
- the oligothiophene derivative of the formula I, wherein X = Y = H and wherein R is b and n = 1.
- the oligothiophene derivative of the formula I, wherein X = Y = H and wherein R is c and n = 1.
- the oligothiophene derivative of the formula I, wherein X = Y = CH3 and wherein R is a and n = 1.
- the oligothiophene derivative of the formula I, wherein X = Y = CH3 and wherein R is b and n = 1.
- the oligothiophene derivative of the formula I, wherein X = Y = CH3 and wherein R is c and n = 1.
- the oligothiophene derivative of the formula I, wherein X = H and Y = CH3 and R is a and n = 1.
- the oligothiophene derivative of the formula I, wherein X = H and Y = CH3 and R is b and n = 1.
- the oligothiophene derivative of the formula I, wherein X = H and Y = CH3 and R is c and n = 1.
- the oligothiophene derivative of the formula I, wherein X = Y = H and wherein R is a and n = 2.
- the oligothiophene derivative of the formula I, wherein X = Y = H and wherein R is b and n = 2.
- the oligothiophene derivative of the formula I, wherein X = Y = H and wherein R is c and n = 2.
- the oligothiophene derivative of the formula I, wherein X = Y = CH₃ and wherein R is a and n = 2.
- the oligothiophene derivative of the formula I, wherein X = Y = CH₃ and wherein R is b and n = 2.
- the oligothiophene derivative of the formula I, wherein X = Y = CH₃ and wherein R is c and n = 2.
- the oligothiophene derivative of the formula I, wherein X = H and Y = CH₃ and R is a and n = 2.
- the oligothiophene derivative of the formula I, wherein X = H and Y = CH₃ and R is b and n = 2.
- the oligothiophene derivative of the formula I, wherein X = H and Y = CH₃ and R is c and n = 2.
- the oligothiophene derivative of formula II, wherein R is a and n = 2.
- the oligothiophene derivative of formula II, wherein R is b and n = 2.
- the oligothiophene derivative of formula II, wherein R is c and n = 2.
- the oligothiophene derivative of formula II, wherein R is a and n = 1.
- the oligothiophene derivative of formula II, wherein R is b and n = 1.
- the oligothiophene derivative of formula II, wherein R is c and n = 1.

The present invention further discloses the use of the oligothiophene derivative of Claim 1 in a liquid crystal medium.

The present invention additionally discloses the use of the oligothiophene derivative of Claim 1 as semiconductor.

The present invention finally discloses an electronic device element comprising the oligothiophene derivative of Claim 1.

### Brief description of the drawings

Fig. 1 represents a photograph of the optical texture of 1,2-di-(5-hexyl-2-terthiophene)ethane 10a at 193°C under polarized light.

Fig. 2 schematically represents the synthesis of the oligothiophene derivatives of the formula II.

Fig. 3 schematically represents the synthesis of the oligothiophene derivatives of the structure I.

Fig. 4 schematically represents the advantage of a bridged molecule compared to a non-bridged molecule.

### Detailed description of the invention

In the different synthetic steps used for the molecules of the present invention, all chemicals were purchased from Aldrich or Acros and used without further purification unless stated otherwise. THF was refluxed over sodium and benzophenone until blue-violet color persisted and directly distilled into a reaction flask. Commercially available solution of BuLi in hexane was titrated with Ph₂CHCOOH immediately before use. *t*-BuOK was used as solution in THF (1M).

For the identification of the chemical structure, ¹H-NMR (300MHz) and ¹³C-NMR (75MHz) spectra were recorded in CDCl₃ on Brucker Avance 300 spectrometer; chemical shifts (δ) are given in ppm relative to TMS (internal standard); coupling constants (*J*) are given in Hz. EI-MS (70 eV) spectra were recorded on a VG Micromass 7070F instrument.

Concerning the liquid crystal properties, phase transition temperatures were measured by differential scanning calorimetry (Mettler Toledo DSC 821), optical textures were observed with polarizing microscopy (Nikon Eclipse 80i). 1,2-Di-(5-bromo-2-thienyl) ethane^{[1]}, 5-hexylterthiophene^{[2]}, 5-(3,7-dimethyloctyl)bithiophene^{[3]} and 5-mercapto-5'-hexylterthiophene^{[4]} were prepared as described in the literature.

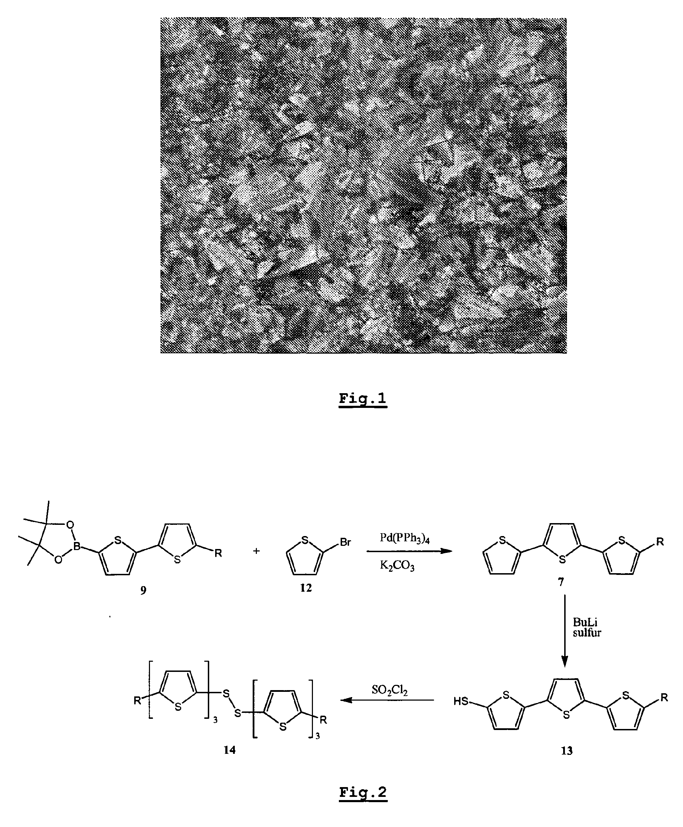

In the following description, the underlined numbers refer to the intermediate molecules shown in Fig. 2 and 3 and the notation a, b and c to the type of R groups as described in Claim 2.

### General procedure 1 for boronic ester derivative

A solution of 1.3mmol of monoalkylated oligothiophene in THF is cooled down to -78°C, 2 eq of BuLi is added dropwise. The mixture is stirred during 15 min. at -78°C. Then 1.6mmol of 2-isopropoxy-4,4-tertramethyl-1,2,3-dioxaborolane is added to the mixture. This solution is stirred during 30 min. at -78°C and slowly heated to room temperature for 3 hours. The reaction mixture is poured into ether (50ml) and then into water mixed with 2.5ml of 1M HCl solution. The organic phase is extracted, dried over MgSO₄ and evaporated. The product is then used for the next reaction without other purification.

### 5-(3,7-dimethyloctyl)-5'-(4,4,5,5-tetramethyl-1,2,3-dioxaborolane-2-yl)-2,2'-bithiophene 8b:

Prepared according to the procedure 1 from 1.3mmol (0.4mg) of 5-(3,7-dimethyloctyl)bithiophene 6b and 0.3ml of 2-isopropoxy-4,4-tertramethyl-1,2,3-dioxaborolane.
A transparent liquid is obtained. Yield: 98%.
¹H NMR (300 MHz, CDCl₃, 25 °C): δ = 7.50 (d, 1H, *J*=3.7Hz); 7.15 (d, 1H, *J*=3.7Hz); 7.03 (d, 1H, *J*=3.7 Hz); 6.68 (d, 1H, *J*=3.7 Hz); 2.80 (t, 2H, *J*=7.7 Hz), 1.71-1.10 (m, 23H), 0.91 (d, 3H, *J*=6.3Hz),0.86 (d, 6H, *J*=6.7Hz) ppm.

### 5-(3,7,11-trimethyldodecyl-2,6,10-triene)-5'-(4,4,5,5-tetramethyl-1,2,3-dioxaborolane-2-yl)-2,2'-bithiophene 8c:

Prepared according to the procedure 1 from 1.8mmol (670mg) of 5-(3,7,11-trimethyldodecyl-2,6,10-triene)bithiophene and 0.5ml of 2-isopropoxy-4,4-tertramethyl-1,2,3-dioxaborolane. A green liquid is obtained. Yield: 98%.
¹H NMR (300 MHz, CDCl₃, 25 °C): δ = 7.50 (d, 1H, *J*=3.7Hz); 7.15 (d, 1H, *J*=3.7Hz); 7.03 (d, 1H, *J*=3.7 Hz); 6,68 (d, 1H, *J*=3.7 Hz); 5.4 (t, *J*=8.8 Hz, 1H), 5.11-5.07 (m, 4H), 3.5 (d, 7.2 Hz, 2H), 1.74-0.8 (m, 30H) ppm.

### 5-hexyl-5"-(4,4,5,5-tetramethyl-1,2,3-dioxaborolane-2-yl)-2,2"-terthiophene 9a:

Prepared according to the procedure 1 from 1.3mmol (440mg) of 5-hexylterthiophene 7a and 1.6mmol (0.4ml) 2-isopropoxy-4,4-tertramethyl-1,2,3-dioxaborolane.
A green solid is obtained. Yield: 96%.
¹H NMR (300 MHz, CDCl₃, 25 °C): δ = 7.51 (d, 1H, *J*=3.7Hz), 7.21 (d, 1H, *J*=3.7Hz), 7.00-6.97 (m, 3H), 6.68(d, 1H, *J*=3.7Hz), 2.79 (t, 2H, *J*=7.3Hz), 1.68 (quint, 4H, *J*=7.7Hz), 1.41-1.24 (m, 15H), 0.89 (t, 3H, *J*=6.6Hz) ppm.

### General procedure 2 for the Suzuki coupling

[0025] 0.7mmol of 1,2-di-(5-bromo-2-thienyl)ethane 4 and 95mg of Pd cat in solution with toluene (10ml) is heated to 75°C, a solution of boron ester (3 eq) in EtOH (6ml) and a solution of K₂CO₃ (18 eq) in water (6ml) are added dropwise. After 24h of stirring at 75°C, the mixture is cooled down and filtered. The solid is cristallised from toluene.

### 1,2-di-(5-hexylterthiophene)ethane 10a:

Prepared according to the general procedure 2 from 300mg of 1,2-di-(5-bromo-2-thienyl)ethane 4 and 905mg of 5-hexyl-5'-(4,4,5,5-tetramethyl-1,2,3-dioxaborolane-2-yl)-2,2'-bithiophene 8a.
An orange solid is obtained after cristallisation from toluene. Yield: 67%.
¹H NMR (300 MHz, CDCl₃, 25 °C): δ = 6.99-6.96 (m, 4H), 6.70 (d, 1H, *J*=3.7Hz), 6.67 (d, 1H, *J*=3.7Hz), 3.17 (s, 4H), 2.79 (t, 4H, *J*=7.3Hz), 1.68 (quint, 4H, *J*=7.3Hz), 1.30-1.33 (m, 12H), 0.89 (t, 6H, *J*=6.9Hz) ppm. DSC: K 113°C LC1 175°C LC2 213°C I.

### 1,2-di-(5-(3,7-dimethyloctyl)terthiophene)ethane 10b:

Prepared according to the general procedure 1 from 0.2mmol (74mg) of 1,2-di-(5-bromo-2-thienyl)ethane 4 and 0.6mmol (250mg) 5-(3,7-dimethyloctyl)-5'-(4,4,5,5-tetramethyl-1,2,3-dioxaborolane-2-yl)-2,2'-bithiophene 9b.
An orange solid is obtained after cristallisation from toluene. Yield: 71%.
¹H NMR (300 MHz, CDCl₃, 25 °C): δ = 6. 99-6. 96 (m, 4H), 6.71 (d, 1H, *J*=3.7Hz); 6.68 (d, 1H, *J*=3.7Hz), 3.17 (s, 4H), 2.79 (t, 4H, *J*=7.5Hz), 1.68 (quint, 4H, *J*=7.5Hz), 1.71-1.10 (m, 16H), 0.91 (d, 6H, J=6.3Hz), 0.86 (d, 12H, J=6.7Hz) ppm. DSC: K 126°C LC 157°C I.

### 1,2-di-(5-hexylquaterthiophene)ethane 11a:

Prepared according to the general procedure 1 from 0.4mmol (147mg) of 1,2-di-(5-bromo-2-thienyl)ethane 4 and 1.2mmol (552mg) 5-hexyl-5"-(4,4,5,5-tetramethyl-1,2,3-dioxaborolane-2-yl)-2,2"-terthiophene 9a.
A red solid is soxhlet in methanol. Yield: 65%.
¹H NMR (300 MHz, CDCl₃, 25 °C): δ = 7.06-6.90 (m, 6H), 6. 68 (d, 1H, *J*=3.3 Hz), 6.64 (d, 1H, *J*=3.3Hz), 3.19 (s, 4H), 2.80 (t, 4H, *J*=7,7Hz), 1.70 (quint, 4H, *J*=7.3Hz), 1.36-1.24 (m, 12H), 0.93 (t, 6H, *J*=6.6Hz) ppm. Mp: 261°C.

### 1,2-di-(5-(3,7,11-trimethyldodecyl-2,6,10-triene)terthiophene)ethane 10c:

Prepared according to the general procedure 1 from 0.2mmol (74mg) of 1,2-di-(5-bromo-2-thienyl)ethane 4 and 0.2mmol (100mg) 5-(3,7,11-trimethyldodecyl-2,6,10-triene)-5'-(4,4,5,5-tetramethyl-1,2,3-dioxaborolane-2-yl)-2,2'-bithiophene 9b.
A yellow solid is obtained after cristallisation from toluene. Yield: 20%.
¹H NMR (300 MHz, CDCl₃, 25 °C): δ = 6.99-6.96 (m, 4H), 6.70 (d, 1H, *J*=3.7Hz), 6.67 (d, 1H, *J*=3.7Hz), 3.17 (s, 4H), 5.4 (t, *J*=6.6Hz, 2H), 5.11-5.09 (m, 8H), 3.5 (d, *J*=6.3 Hz, 4H), 2.33-1.94 (m, 8H), 1.61 (s, 6H), 1.59 (s, 6H), 1.45-1.33 (m, 4H), 1.08 (s, 6H), 1.06 (s, 6H) ppm.

### 1,2-di-(5-(3,7-dimethyloctyl)-5-terthiophene)disulfide 14a:

0.04my of SO₂Cl₂ in 4ml of dichloromethane were added dropwise to 0.8mmol (300mg) of 5-mercapto-5'-hexylterthiophene 13a in solution in 2ml of dichloromethane at room temperature. The solution is stirred for 1 hour at room temperature. After completion of the reaction, the solvent is evaporated in vacuum.
A yellow solid is obtained. Yield: 85%.
¹H NMR (300 MHz, CDCl₃, 25 °C) : δ = 7.09 (t, 2H, *J*=4.0Hz), 7.04-6.99 (m, 3H), 6.68 (d, 1H, *J*=3.7 Hz), 2.80 (t, 2H, *J*=7.3Hz), 1.68 (quint, 4H, *J*=7.3Hz), 1.41-1.25 (m, 28H), 0.89 (t, 6H, *J*=6.6Hz)ppm. Decomposition > 250°C.

## Claims

1. An oligothiophene derivative of the formula I
or II: 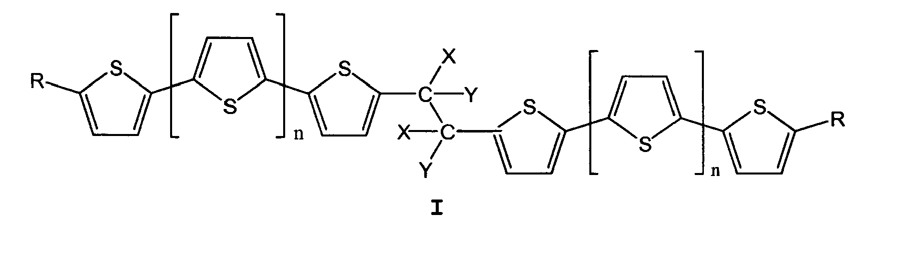 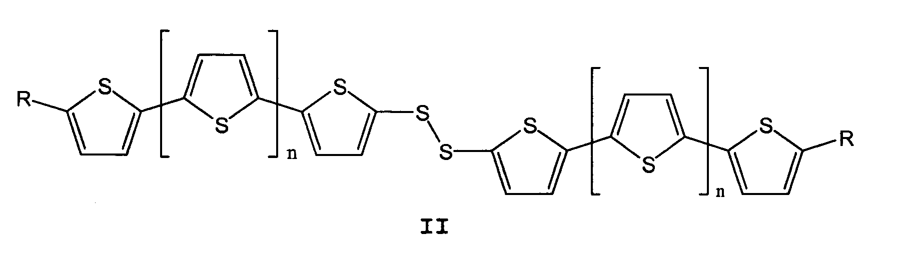 wherein
X = H or CH₃,
Y = H or CH₃,
n = 1 or 2, and
R = carbon chain, branched or not, saturated or not, with 6 to 15 carbon atoms.

2. The oligothiophene derivative of Claim 1
wherein R is a, b, or c with:
a : 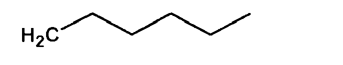
b : 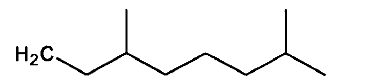
c :

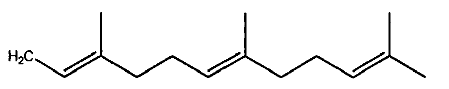

3. The oligothiophene derivative of Claim 1 wherein said derivative is formula I, wherein X = Y = H and wherein R is a and n = 1.

4. The oligothiophene derivative of Claim 1 wherein said derivative is formula I, wherein X = Y = H and wherein R is b and n = 1.

5. The oligothiophene derivative of Claim 1 wherein said derivative is formula I, wherein X = Y = H and wherein R is c and n = 1.

6. The oligothiophene derivative of Claim 1 wherein said derivative is formula I, wherein X = Y = CH₃ and wherein R is a and n = 1.

7. The oligothiophene derivative of Claim 1 wherein said derivative is formula I, wherein X = Y = CH₃ and wherein R is b and n = 1.

8. The oligothiophene derivative of Claim 1 wherein said derivative is formula I, wherein X = Y = CH₃ and wherein R is c and n = 1.

9. The oligothiophene derivative of Claim 1 wherein said derivative is formula I and wherein X = H and Y = CH₃ and R is a and n = 1.

10. The oligothiophene derivative of Claim 1 wherein said derivative is formula I and wherein X = H and Y = CH₃ and R is b and n = 1.

11. The oligothiophene derivative of Claim 1 wherein said derivative is formula I and wherein X = H and Y = CH₃ and R is c and n = 1.

12. The oligothiophene derivative of Claim 1 wherein said derivative is formula I, wherein X = Y = H and wherein R is a and n = 2.

13. The oligothiophene derivative of Claim 1 wherein said derivative is formula I, wherein X = Y = H and wherein R is b and n = 2.

14. The oligothiophene derivative of Claim 1 wherein said derivative is formula I, wherein X = Y = H and wherein R is c and n = 2.

15. The oligothiophene derivative of Claim 1 wherein said derivative is formula I, wherein X = Y = CH₃ and wherein R is a and n = 2.

16. The oligothiophene derivative of Claim 1 wherein said derivative is formula I, wherein X = Y = CH₃ and wherein R is b and n = 2.

17. The oligothiophene derivative of Claim 1 wherein said derivative is formula I, wherein X = Y = CH₃ and wherein R is c and n = 2.

18. The oligothiophene derivative of Claim 1 wherein said derivative is formula I and wherein X = H and Y = CH₃ and R is a and n = 2.

19. The oligothiophene derivative of Claim 1 wherein said derivative is formula I and wherein X = H and Y = CH₃ and R is b and n = 2.

20. The oligothiophene derivative of Claim 1 wherein said derivative is formula I and wherein X = H and Y = CH₃ and R is c and n = 2.

21. The oligothiophene derivative of Claim 1 wherein said derivative is formula II and wherein R is a and n = 2.

22. The oligothiophene derivative of Claim 1 wherein said derivative is formula II, and wherein R is b and n = 2.

23. The oligothiophene derivative of Claim 1 wherein said derivative is formula II, and wherein R is c and n = 2.

24. The oligothiophene derivative of Claim 1 wherein said derivative is formula II, and wherein R is a and n = 1.

25. The oligothiophene derivative of Claim 1 wherein said derivative is formula II, and wherein R is b and n = 1.

26. The oligothiophene derivative of Claim 1 wherein said derivative is formula II, and wherein R is c and n = 1.

27. Use of the oligothiophene derivative of Claim 1 in a liquid crystal medium.

28. Use of the oligothiophene derivative of Claim 1 as semiconductor.

29. Electronic device element comprising the oligothiophene derivative of Claim 1.

## Patentansprüche

1. Oligothiophenderivat der Formel I oder II: 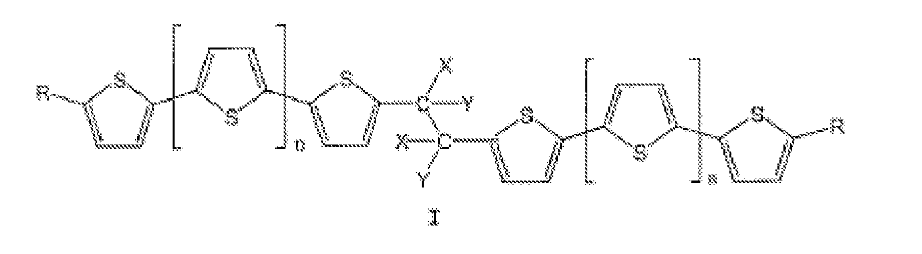 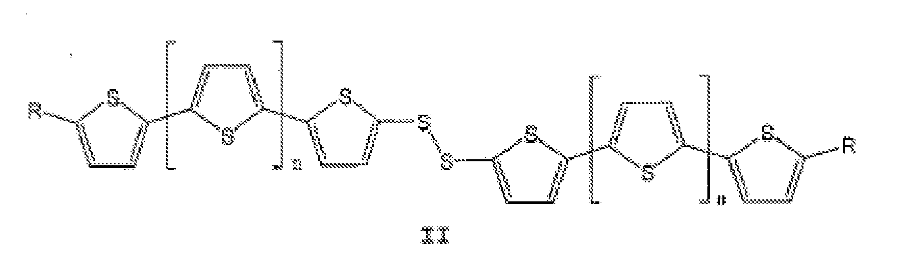 wobei
X = H oder CH₃,
Y = H oder CH₃,
n = 1 oder 2, und
R = Kohlenstoffkette, verzweigt oder nicht, gesättigt oder nicht, mit 6 bis 15 Kohlenstoffatomen.

2. Oligothiophenderivat nach Anspruch 1, wobei R a, b oder c ist, wobei:
a: 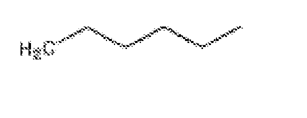
b : 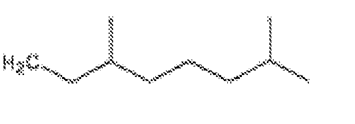
c :

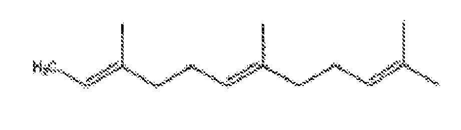

3. Oligothiophenderivat nach Anspruch 1, wobei das Derivat Formel I ist, wobei X = Y = H, und wobei R a ist und n = 1.

4. Oligothiophenderivat nach Anspruch 1, wobei das Derivat Formel I ist, wobei X = Y = H, und wobei R b ist und n = 1.

5. Oligothiophenderivat nach Anspruch 1, wobei das Derivat Formel I ist, wobei X = Y = H, und wobei R c ist und n = 1.

6. Oligothiophenderivat nach Anspruch 1, wobei das Derivat Formel I ist, wobei X = Y = CH₃, und wobei R a ist und n = 1.

7. Oligothiophenderivat nach Anspruch 1, wobei das Derivat Formel I ist, wobei X = Y = CH₃, und wobei R b ist und n = 1.

8. Oligothiophenderivat nach Anspruch 1, wobei das Derivat Formel I ist, wobei X = Y = CH₃, und wobei R c ist und n = 1.

9. Oligothiophenderivat nach Anspruch 1, wobei das Derivat Formel I ist, und wobei X = H und Y = CH₃ und R a ist und n = 1.

10. Oligothiophenderivat nach Anspruch 1, wobei das Derivat Formel I ist, und wobei X = H und Y = CH₃ und R b ist und n = 1.

11. Oligothiophenderivat nach Anspruch 1, wobei das Derivat Formel I ist, und wobei X = H und Y = CH₃ und R c ist und n = 1.

12. Oligothiophenderivat nach Anspruch 1, wobei das Derivat Formel I ist, wobei X = Y = H, und wobei R a ist und n = 2.

13. Oligothiophenderivat nach Anspruch 1, wobei das Derivat Formel I ist, wobei X = Y = H, und wobei R b ist und n = 2.

14. Oligothiophenderivat nach Anspruch 1, wobei das Derivat Formel I ist, wobei X = Y = H, und wobei R c ist und n = 2.

15. Oligothiophenderivat nach Anspruch 1, wobei das Derivat Formel I ist, wobei X = Y = CH₃, und wobei R a ist und n = 2.

16. Oligothiophenderivat nach Anspruch 1, wobei das Derivat Formel I ist, wobei X = Y = CH₃, und wobei R b ist und n = 2.

17. Oligothiophenderivat nach Anspruch 1, wobei das Derivat Formel I ist, wobei X = Y = CH₃, und wobei R c ist und n = 2.

18. Oligothiophenderivat nach Anspruch 1, wobei das Derivat Formel I ist, und wobei X = H und Y = CH₃ und R a ist und n = 2.

19. Oligothiophenderivat nach Anspruch 1, wobei das Derivat Formel I ist, und wobei X = H und Y = CH₃ und R b ist und n = 2.

20. Oligothiophenderivat nach Anspruch 1, wobei das Derivat Formel I ist, und wobei X = H und Y = CH₃ und R c ist und n = 2.

21. Oligothiophenderivat nach Anspruch 1, wobei das Derivat Formel II ist, und wobei R a ist und n = 2.

22. Oligothiophenderivat nach Anspruch 1, wobei das Derivat Formel II ist, und wobei R b ist und n = 2.

23. Oligothiophenderivat nach Anspruch 1, wobei das Derivat Formel II ist, und wobei R c ist und n = 2.

24. Oligothiophenderivat nach Anspruch 1, wobei das Derivat Formel II ist, und wobei R a ist und n = 1.

25. Oligothiophenderivat nach Anspruch 1, wobei das Derivat Formel II ist, und wobei R b ist und n = 1.

26. Oligothiophenderivat nach Anspruch 1, wobei das Derivat Formel II ist, und wobei R c ist und n = 1.

27. Verwendung des Oligothiophenderivats nach Anspruch 1 in einem Flüssigkristallmedium.

28. Verwendung des Oligothiophenderivats nach Anspruch 1 als Halbleiter.

29. Elektronisches Vorrichtungselement, umfassend das Oligothiophenderivat nach Anspruch 1.

## Revendications

1. Dérivé d'oligothiophène de formule I ou II : 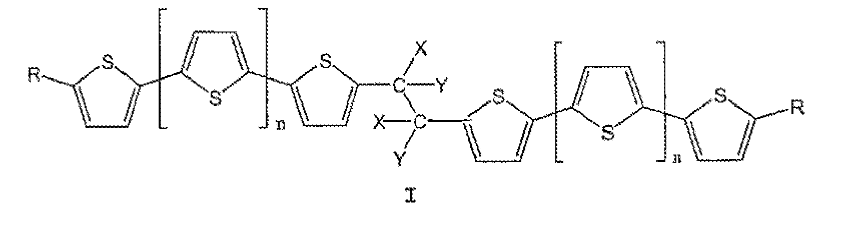 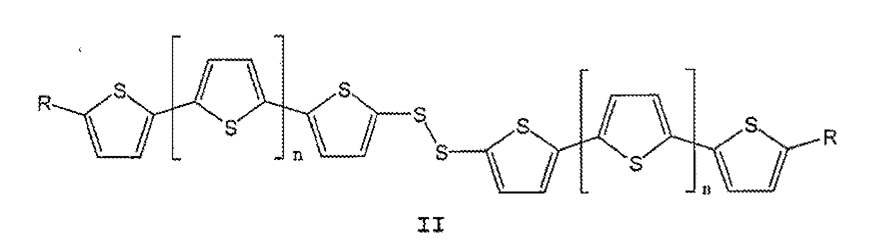 où
X = H ou CH₃,
Y = H ou CH₃,
n = 1 ou 2, et
R = chaîne carbonée, ramifiée ou non ramifiée, saturée ou insaturée, ayant de 6 à 15 atomes de carbone.

2. Dérivé d'oligothiophène selon la revendication 1, dans lequel R est a, b ou c, où :
**a :** 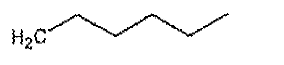
**b :** 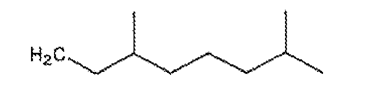
**c :**

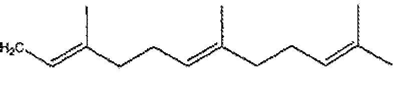

3. Dérivé d'oligothiophène selon la revendication 1, lequel dérivé est de formule I dans laquelle X = Y = H et dans laquelle R est a et n = 1.

4. Dérivé d'oligothiophène selon la revendication 1, lequel dérivé est de formule I dans laquelle X = Y = H et dans laquelle R est b et n = 1.

5. Dérivé d'oligothiophène selon la revendication 1, lequel dérivé est de formule I dans laquelle X = Y = H et dans laquelle R est c et n = 1.

6. Dérivé d'oligothiophène selon la revendication 1, lequel dérivé est de formule I dans laquelle X = Y = CH₃ et dans laquelle R est a et n = 1.

7. Dérivé d'oligothiophène selon la revendication 1, lequel dérivé est de formule I dans laquelle X = Y = CH₃ et dans laquelle R est b et n = 1.

8. Dérivé d'oligothiophène selon la revendication 1, lequel dérivé est de formule I dans laquelle X = Y = CH₃ et dans laquelle R est c et n = 1.

9. Dérivé d'oligothiophène selon la revendication 1, lequel dérivé est de formule I dans laquelle X = H et Y = CH₃ et dans laquelle R est a et n = 1.

10. Dérivé d'oligothiophène selon la revendication 1, lequel dérivé est de formule I dans laquelle X = H et Y = CH₃ et dans laquelle R est b et n = 1.

11. Dérivé d'oligothiophène selon la revendication 1, lequel dérivé est de formule I dans laquelle X = H et Y = CH₃ et dans laquelle R est c et n = 1.

12. Dérivé d'oligothiophène selon la revendication 1, lequel dérivé est de formule I dans laquelle X = Y = H et dans laquelle R est a et n = 2.

13. Dérivé d'oligothiophène selon la revendication 1, lequel dérivé est de formule I dans laquelle X = Y = H et dans laquelle R est b et n = 2.

14. Dérivé d'oligothiophène selon la revendication 1, lequel dérivé est de formule I dans laquelle X = Y = H et dans laquelle R est c et n = 2.

15. Dérivé d'oligothiophène selon la revendication 1, lequel dérivé est de formule I dans laquelle X = Y = CH₃ et dans laquelle R est a et n = 2.

16. Dérivé d'oligothiophène selon la revendication 1, lequel dérivé est de formule I dans laquelle X = Y = CH₃ et dans laquelle R est b et n = 2.

17. Dérivé d'oligothiophène selon la revendication 1, lequel dérivé est de formule I dans laquelle X = Y = CH₃ et dans laquelle R est c et n = 2.

18. Dérivé d'oligothiophène selon la revendication 1, lequel dérivé est de formule I dans laquelle X = H et Y = CH₃ et dans laquelle R est a et n = 2.

19. Dérivé d'oligothiophène selon la revendication 1, lequel dérivé est de formule I dans laquelle X = H et Y = CH₃ et dans laquelle R est b et n = 2.

20. Dérivé d'oligothiophène selon la revendication 1, lequel dérivé est de formule I dans laquelle X = H et Y = CH₃ et dans laquelle R est c et n = 2.

21. Dérivé d'oligothiophène selon la revendication 1, lequel dérivé est de formule II dans laquelle R est a et n = 2.

22. Dérivé d'oligothiophène selon la revendication 1, lequel dérivé est de formule II dans laquelle R est b et n = 2.

23. Dérivé d'oligothiophène selon la revendication 1, lequel dérivé est de formule II dans laquelle R est c et n = 2.

24. Dérivé d'oligothiophène selon la revendication 1, lequel dérivé est de formule II dans laquelle R est a et n = 1.

25. Dérivé d'oligothiophène selon la revendication 1, lequel dérivé est de formule II dans laquelle R est b et n = 1.

26. Dérivé d'oligothiophène selon la revendication 1, lequel dérivé est de formule II dans laquelle R est c et n = 1.

27. Utilisation du dérivé d'oligothiophène de la revendication 1 dans un milieu cristal liquide.

28. Utilisation du dérivé d'oligothiophène de la revendication 1 comme semi-conducteur.

29. Elément de dispositif électronique comprenant le dérivé d'oligothiophène de la revendication 1.
